(19) Europäisches Patentamt

European Patent Office

Office européen des brevets

(11) EP 0 573 435 B1

(12) **EUROPEAN PATENT SPECIFICATION**

(45) Date of publication and mention
of the grant of the patent:
**29.10.1997 Bulletin 1997/44**

(21) Application number: **92901932.1**

(22) Date of filing: **23.12.1991**

(51) Int Cl.6: **C12N 15/31**, C07K 2/00,
A61K 39/10

(86) International application number:
**PCT/GB91/02302**

(87) International publication number:
**WO 92/11292 (09.07.1992 Gazette 1992/17)**

(54) **ACELLULAR VACCINE**

AZELLULÄRE IMPFSTOFFE

VACCIN ACELLULAIRE

(84) Designated Contracting States:
**AT BE CH DE ES FR GB IT LI NL**

(30) Priority: **21.12.1990 GB 9027901**

(43) Date of publication of application:
**15.12.1993 Bulletin 1993/50**

(60) Divisional application: **97104861.6**

(73) Proprietor: **EVANS MEDICAL LIMITED
Leatherhead, Surrey KT22 7PQ (GB)**

(72) Inventors:
- **CHARLES, Ian George
  Beckenham, Kent BR3 3BS (GB)**
- **Dougan, Gordon Dept. of Biochemistry
  LONDON SW7 2AZ (GB)**
- **Li, Jing Li Dept. of Biochemistry
  LONDON SW7 2AZ (GB)**

(74) Representative: **Woods, Geoffrey Corlett et al
J.A. KEMP & CO.
14 South Square
Gray's Inn
London WC1R 5LX (GB)**

(56) References cited:
**EP-A- 0 425 082          WO-A-91/15571**

- **DIALOG INFORMATION SERVICES, File 154,
  Medline 85-92; L.J. LI et al., Dialog AN 07732771,
  Medline AN 91251771**
- **BIOTECHNOLOGY, vol. 8, November 1990; A.J.
  MAKOFF et al., pp. 1030-1033**
- **PROCEEDINGS OF THE NATL. ACADEMY OF
  SCIENCES USA, vol. 86, May 1989, Washington,
  DC (US); I.G. CHARLES et al., pp. 3554-3558**

## Description

The invention relates to the isolation and characterisation of an antigen of Bordetella parapertussis, DNA sequences encoding the antigen, expression vectors containing such DNA sequences, host cells transformed by such expression vectors. The invention also relates to the use of the antigen in a vaccine for the prevention of B.parapertussis infections.

B.pertussis causes a serious and debilitating disease in humans, especially children. It has been kept under control in the developed countries by large scale immunisation programmes. Traditionally, immunisation has been carried out using a whole cell vaccine derived from cell cultures of B.pertussis and has been found to be relatively effective in preventing the disease.

Concern over adverse reactions, such as fever, local reactions and persistent screaming, has led in recent years to a reduced acceptance of the whole cell B.pertussis vaccine and debate about its continued use. Research has therefore been directed towards the development of vaccines which lack the components responsible for the adverse effects of the whole cell vaccines, whilst retaining the components necessary to confer protection against the disease.

The search for safe and effective vaccines has been hampered by the paucity of information regarding the identity and mechanisms of action of the pathogenic, toxic and protective moieties of the bacterial organism contained in whole cell vaccines. The search for effective vaccines has, therefore, focused on the isolation and characterisation of surface antigens of the organism and on their efficacy in inducing an immune reaction (J. Am.Med.Soc., 1982, 248(1) 22-23). Examples of B.pertussis antigens that have been investigated include lymphocytosis promoting factor (LPF, otherwise known as pertussis toxin (PT)), filamentous haemagglutinin (FHA), lipopolysaccharide (LPS), agglutinogens, dermonecrotic toxin (DNT), heat labile and heat stable toxins, polymorphonuclear leukocyte inhibitor factor, adenylate cyclase, the outer membrane 69kDa antigen (P. 69, pertactin) and other surface components.

B.parapertussis is closely related to B.pertussis and is also responsible for outbreaks of whooping cough in man (Zeulzer et al, J. Pediatr. 9:493-497 (1946); Linneman et al J. Pediatr. 19: 229-240 (1977); Novotny, J.Infect. Dis. 161:581-582, 1990)). However, to date no vaccine specific for B.parapertussis has been developed.

B.parapertussis is also known to produce a number of virulence factors (Pitmann, Bergey's Manual of Systematic Bacteriology Vol I P388-393 Ed. Kreig & Hoft 1984) including FHA, and adenylate cyclase (AdeCase) toxin. Because of the immunological cross-reactivity of these virulence factors, vaccination against whooping cough with a whole cell vaccine may provide some protection against B.parapertussis. However, B.parapertussis does not produce the well characterised pertussis toxin (PT). Thus, outbreaks of B.parapertussis mediated whooping cough are unlikely to be prevented by acellular vaccines composed primarily of detoxified pertussis toxin (PT).

Furthermore, if vaccination programmes against B.pertussis are successful in reducing the incidence of the bacterial organism in the human population, or even in eradicating the organism altogether, just as smallpox has been eradicated by rigorous vaccination programmes, there is a danger that B.parapertussis infections will increase in number and B.parapertussis will become the primary cause of whooping cough. It is therefore desirable to include in any whooping cough vaccination programme, a B.parapertussis specific vaccine, preferably an acellular vaccine.

I.G. Charles et al Proc. Natl. Acad Sci USA Vol 80 pp 3554-3558 (1989) purport to identify homologous proteins in B.pertussis and B.parapertussis with molecular masses of 69 and 70kDa respectively. The assertion of homology is based on the observation that these antigens bind to the BB05 antibody raised against B. bronchiseptica. These proteins although clearly related to each other in their ability to bind BB05 antibody have different immunogenic properties. It is not stated whether the antigen of B.parapertussis would be expected to have any immunogenic potential in vivo, this being necessary for use as an effective vaccine.

The inventors have identified, isolated and characterised an antigen of B.parapertussis described hereinafter, which is useful as the basis for an acellular vaccine specific for B.parapertussis or as part of an acellular whooping cough vaccine.

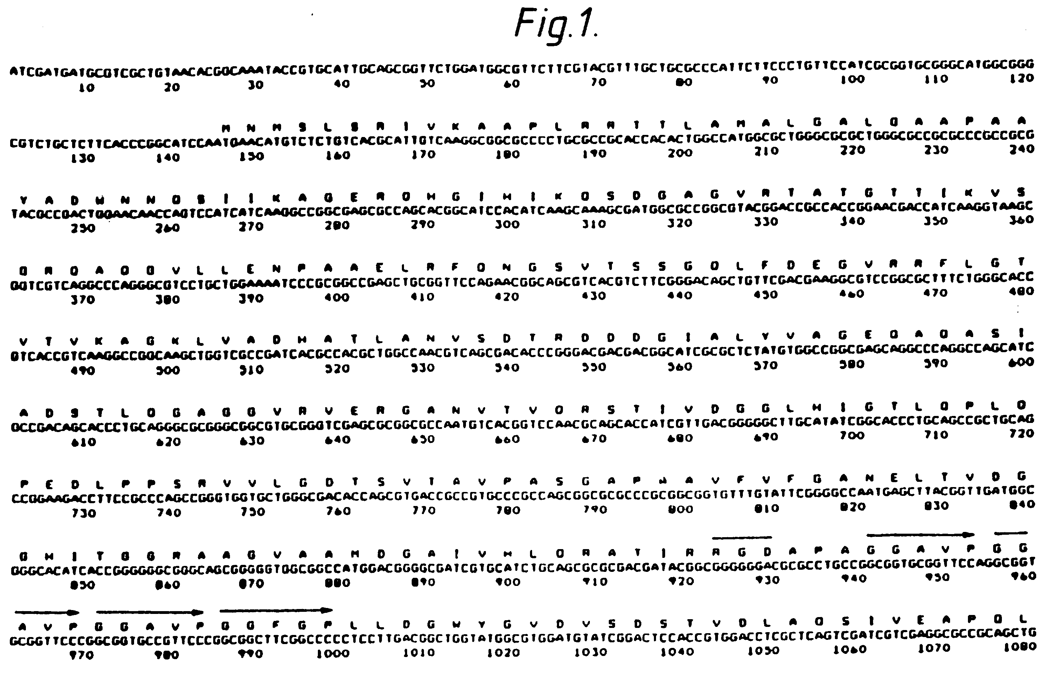

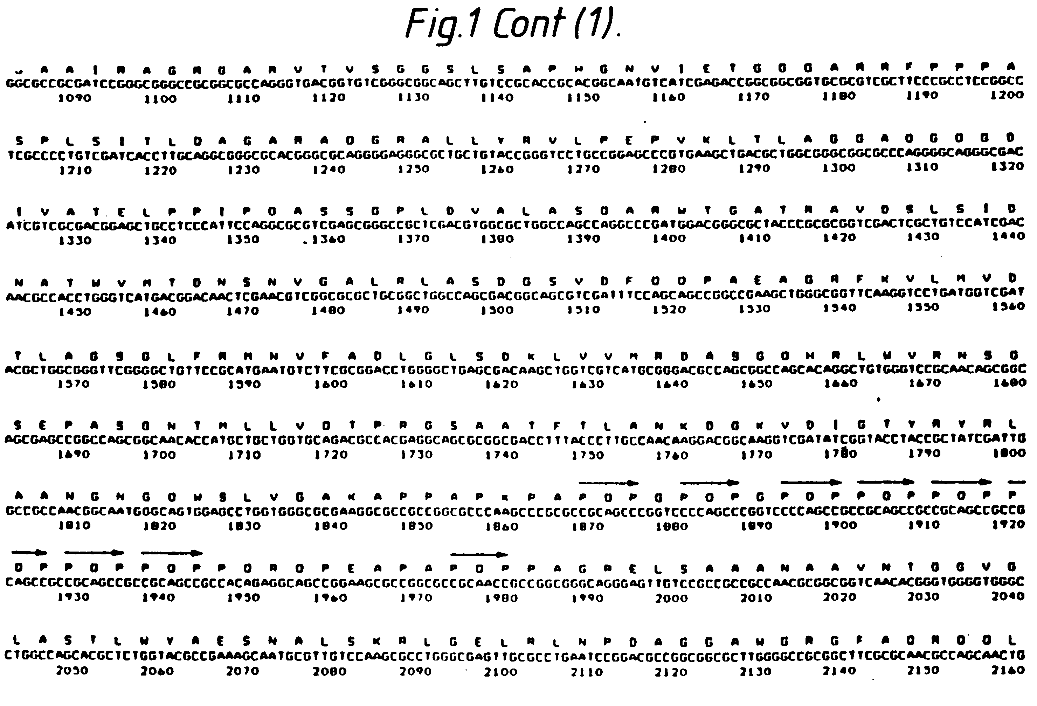

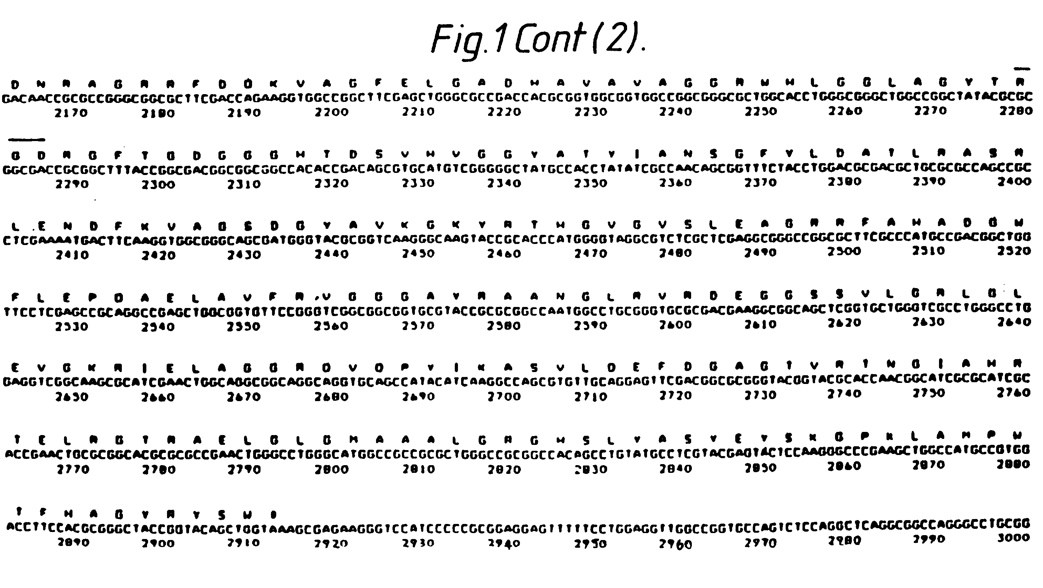

The invention provides a protein which is uncontaminated by components from B.parapertussis, which is capable of binding to antibody which also binds the native P70 antigen of B.parapertussis and which has (a) the amino acid sequence shown in Figure 1 from amino acid residue Asp 35 to Asn 643, or (b) an amino acid sequence which has a homology of more than 98% with the said amino acid sequence (a).

The invention also provides a DNA sequence which encodes this protein. Such a DNA sequence typically consists essentially of the nucleotide sequence shown in Figure 1 from nucleotide 247 to nucleotide 2073. The DNA sequence may be a sequence which differs from the nucleotide sequence shown in Figure 1 from nucleotide 247 to nucleotide 2073 at no more than twelve positions.

The invention further provides a protein which has the amino acid sequence shown in Figure 1, or an amino acid sequence which has a homology of more than 98% with the amino acid sequence shown in Figure 1.

Also provided is a DNA sequence which encodes this protein. Such a DNA sequence typically consists essentially of the nucleotide sequence shown in Figure 1 from nucleotide 145 to nucleotide 2910. The DNA sequence may be a sequence which differs from the nucleotide sequence shown in Figure 1 from nucleotide 145 to nucleotide 2910 at no more than twelve positions.

The amino acid sequence of Figure 1 shows the precursor protein of molecular weight approximately 95,000 Daltons (P.95) comprising 922 amino acids. *In vivo* processing of this precursor yields a protein with an estimated molecular weight of 70,000 Daltons (P.70) as determined by SDS polyacrylamide gel electrophoresis, but the actual weight is approximately 61 kDa.

Antigenic sites of the amino acid sequence set out in Figure 1 may be identified using standard procedures. These may involve fragmentation of the polypeptide itself using proteolytic enzymes or chemical agents and then determining the ability of each fragment to bind to antibodies or to provide an immune response when innoculated into an animal or suitable in vitro model system (Strohmauer et al, J. Gen.Virol, 1982, 59, 205-306). Alternatively, the DNA encoding the polypeptide may be fragmented by restriction enzyme digestion or other well-known techniques and then introduced into an expression system to produce fragments (optionally fused to a polypeptide usually of bacterial origin). The resulting fragments are assessed as described previously (Spence et al, J.Gen.Virol., 1989, 70, 2843-51; Smith et al, Gene, 1984, 29, 263-9). Another approach is to chemically synthesise short peptide fragments (3-20 amino acids long; conventionally 6 amino acids long) which cover the entire sequence of the full-length polypeptide with each peptide overlapping the adjacent peptide. (This overlap can be from 1-10 amino acids but ideally is n-1 amino acids where n is the length of the peptide; Geysen et al, Proc. Natl. Acad. Sci., 1984, 81, 3998-4002). Each peptide is then assessed as described previously except that the peptide is usually first coupled to some carrier molecule to facilitate the induction of an immune response. Finally, there are predictive methods which involve analysis of the sequence for particular features, e.g. hydrophilicity, thought to be associated with immunologically important sites (Hopp and Woods, Proc. Natl. Acad. Sci., 1981, 78, 3824-8; Berzofsky, Science, 1985, 229, 932-40). These predictions may then be tested using the recombinant polypeptide or peptide approaches described previously.

The antigen of the present invention is located on the surface of *B.parapertussis,* and may be isolated from cultures of *B.parapertussis* by conventional methods, for example as described by Novotny et al (1985) Infection and Immunity 50, 199-206 and European Publication No. 0162639. Alternatively, it may be obtained using recombinant DNA technology. In this regard, the DNA encoding the antigen may be cloned, for example as described by Makoff et al (Bio-Technology November 1990), inserted into an expression vector which is used to enable expression in an appropriate host. A DNA sequence of the invention may thus be a cloned sequence.

The invention also provides a DNA sequence which consists essentially of the sequence shown in Figure 1 or a sequence which has a homology of more than 98% with the sequence shown in Figure 1. The DNA sequence encoding the precursor protein is the sequence from nucleotide 145 to nucleotide 2910. The DNA sequence encoding the P70 protein is the sequence from nucleotide 247 to nucleotide 2073.

The antigen is preferably provided in a pure form ie. greater than 90%, most preferably greater than 95%, pure but at least to a level consistent with its use in a human vaccine.

The cloning of the DNA sequence may be carried out using standard procedures known in the art. However, it is particularly advantageous in such procedures to employ the sequence data disclosed herein so as to facilitate the identification and isolation of the desired cloned DNA sequences. Preferably, the DNA is isolated by the method described by Hull et al Infect. Immun. 33: 933-938 (1981) as modified by Maskell et al J. Bacteriol. 170: 2467-2471 (1988). The DNA is then digested with a restriction enzyme to generate short fragments which are then inserted into a cloning vector, such as the cosmid pHC79 or a derivative thereof and the resulting recombinant DNA molecules used to transform E.coli and thus generate the desired library.

The library may be screened using a standard screening strategy. For example one may employ as hybridisation probes one or more labelled oligonucleotides synthesised using the DNA sequence information disclosed herein. One or more additional rounds of screening of one kind or another may be carried out to characterise and identify positive clones.

Having identified a first positive clone, the library may be rescreened for additional positive clones using the first clone as an hybridization probe. Alternatively or additionally, further libraries may be prepared and these may be screened using hybridisation probes. In this way, further DNA sequences may be obtained.

Alternatively, the DNA sequence encoding the antigen of the present invention may be synthesised using standard procedures and this may be preferred to cloning the DNA in some circumstances.

Thus cloned or synthesised, the desired DNA sequence may be inserted into an expression vector using known and standard techniques. The expression vector is normally cut using restriction enzymes and the DNA sequence inserted using blunt-end or staggered-end ligation. The cut is usually made at a restriction site in a convenient position in the expression vector such that, once inserted, the DNA sequence is under the control of the elements of DNA that effect its expression.

These elements may vary according to the host but usually include a promoter, ribosome binding site, translational start and stop sites, and a transcriptional termination site. Examples of such vectors include plasmids and cosmids. Expression vectors of the present invention encompass both extrachromosomal vectors and vectors that are integrated into the host cell's chromosome.

The invention therefore provides an expression vector which contains a DNA sequence of the invention and which, when provided in a suitable host, is capable of expressing a protein of the invention. The vector is typically a plasmid. The invention also provides a host transformed with an expression vector of the invention.

Examples of host cells of use with the invention include prokaryotic and eukaryotic cells, such as bacterial, yeast, mammalian and insect cells. Particular examples of such cells are *E.coli, S.cerevisiae, P.pastoris,* Chinese hamster ovary and mouse cells, and *Spodoptera frugiperda* and *Tricoplusia ni.* The choice of host cell may depend on a number of factors but, if post-translational modification of the antigen is important, then a prokaryotic host would be preferred.

Transformation of a host cell may be carried out using standard techniques. Some phenotypic marker is usually employed to distinguish between the transformants that have successfully taken up the expression vector and those that have not. Culturing of the transformed host cell and isolation of the antigen may also be carried out using standard techniques.

The invention thus provides a process for the preparation of a protein of the invention, which process comprises maintaining a host transformed with an expression vector which contains a DNA sequence encoding the protein and which is capable of expressing the protein in the host, under such conditions that the protein is expressed. An embodiment of the process comprises cloning or synthesising a DNA sequence encoding the protein, inserting the DNA sequence into an expression vector such that it is capable in an appropriate host of being expressed, transforming a host cell with the expression vector, culturing the transformed host cell and isolating the antigen.

The antigen obtained in this way may be insoluble and thus may need to be refolded following the use of guanidinium hydrochloride as denaturant in conventional manner and in any event is preferably purified.

The invention additionally provides a pharmaceutical composition comprising a pharmaceutically acceptable carrier or diluent and, as active ingredient, a protein of the invention. Thus, a vaccine containing an antigen of *B.parapertussis* is provided. The vaccine of the invention may optionally contain additional antigens of *B.parapertussis* or other bacteria, such as *B.pertussis,* tetanus and diphtheria.

The vaccine of the invention is thus normally associated with a pharmaceutically acceptable vehicle which allows the antigen to be administered to the patient. Administration is usually carried out via the oral, intranasal, or preferably parenteral route. In the case of the parenteral route, the vehicle is generally liquid and the antigen is generally dissolved or suspended in it. An example of a liquid vehicle is physiological saline solution.

The vaccine may also contain an adjuvant for stimulating the immune response and thereby enhancing the potency of the antigen. Convenient adjuvants for use in the present invention include, for example, aluminium hydroxide and aluminium phosphate.

Conveniently the vaccine contains a final concentration of antigen in the range of from 0.01 to 5 mg/ml, preferably 0.03 to 2 mg/ml, most preferably 0.3 mg/ml. After formulation the vaccine may be incorporated into a sterile container which is then sealed and stored at a low temperature, for example 4°C, or is freeze-dried.

A method for inducing immunity to whooping cough in humans is thus provided, the method comprising the administration to the patient of an effective amount of the vaccine of the present invention. In order to induce immunity to whooping cough in humans one or more doses of the vaccine are normally administered. Each dose of the vaccine is 0.1 to 2 ml, preferably 0.2 to 1 ml, most preferably 0.5 ml.

The invention will now be exemplified further by reference to the accompanying Figures and the Examples.

Figure Legends

Figure 1. The amino acid sequence of P.70 and the DNA sequence of the prn gene encoding the P.70 antigen from B.parapertussis.

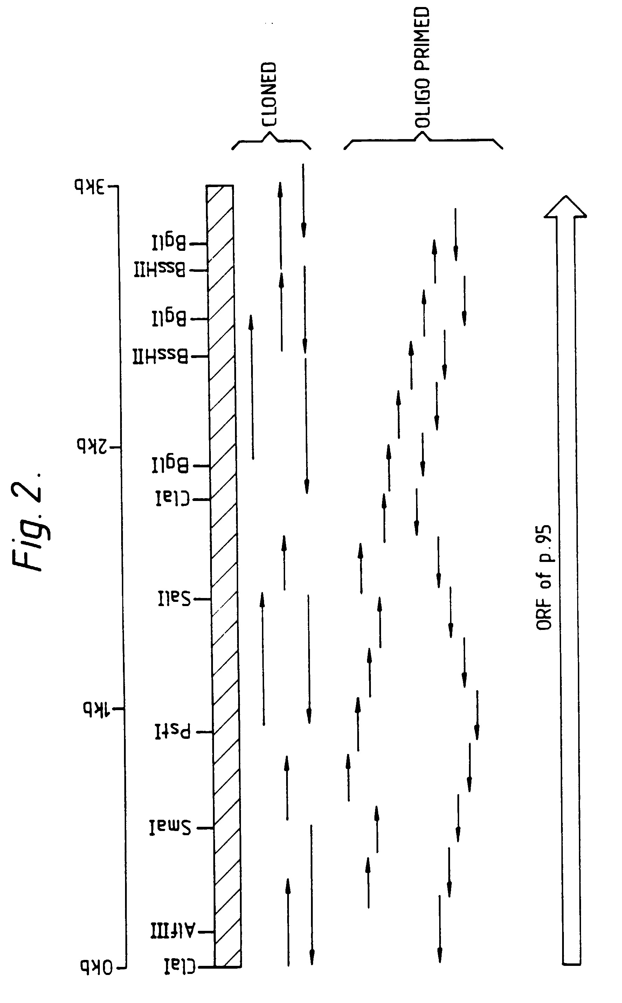

Figure 2. Restriction map and sequencing strategy for the prn gene encoding the P.70 antigen from B.parapertussis. Small arrows represent the direction and extent of DNA sequencing derived from restriction sites or oligonucleotides used as specific primers. The large arrow indicates the direction of transcription of the orf for the gene.

Figure 3. Line drawing showing the strategy used to generate the P.70 antigen expression plasmid pBD845.

Experimental procedures

Bacterial strains, plasmids and phage

B.pertussis strain CN2992, B.parapertussis strain CN2591 were from the Wellcome Culture Collection. E. coli K.12 TG1 [Δ (lac-pro) supE, thi, hsdD5/F' traD36 proA+B+, lacIq lacZ M15 was as described (Carter et al, (1985)] as was E.coli K12 HB101 F⁻, hsdS20 (r⁻B m⁻B) recA13, ara-14, proA2, lacY1, galK2, rpsL20, xyl-5, mtl-1, supE44 (Boyer & Roulland-Dussoix, J.Mol.Biol. 41: 459-465, 1969).

Cosmid pHC79 (Hohn & Collins, Gene 11:29-298, 1980) was from Amersham International, Little Chalfont, Buckinghamshire; plasmid pKK223-2 (Amman & Brosius, Gene 40:183-90, 1985) and M13mp18 and M13mp19 (Yanisch-Perron et al, Gene 33:103-119, 1985) were supplied by Pharmacia Ltd., Milton Keynes, Buckinghamshire.

Media and reagents

E.coli strains were grown on Luria broth (LB) or on LB solidified with 1.6% (w/v) agar (Miller, Experiments in Mol.Gene. Cold Spring Harbor N.Y., 1972) (Difco Address). Minimal medium (MM) was made as described by Miller (1972) and was solidified with 2% (w/v) Noble agar (Difco). B.pertussis and B.parapertussis were grown in Stainer-Scholte broth at 37°C (Stainer & Scholte, J.Gen.Microbiol 63:211-220 1962), or on Stainer-Scholte plates solidified with 2% (w/v) Noble agar. Deoxynucleotides, ampicillin, tetracyline and dithiothreitol were from Sigma. Restriction endonucleases and T4 DNA ligase were from BRL, Paisley, Scotland.

Cloning of B.parapertussis chromosomal DNA into cosmid pHC79 and identification of the gene encoding P.70

B.parapertussis chromosomal DNA (prepared by the method of Hull et al Infect.Immun. 33:933-938, 1981 as modified by Maskell et al J.Bacteriol. 170:2467-2471, 1988) was partially digested with Sau3A, and fragments in the 40-50kb size range were ligated into the BamHI site (Maniatis et al Molecular Cloning: Cold Spring Harbor N.Y, 1982; of cosmid pHC79 (Hohn & Collins Gene 11:291-298, 1980). Recombinant cosmids in E.Coli HB101 were plated out and transferred to microtiter plates following the method described by Charles et al (J.Gen.Microbiol. 136:353-358, 1990) and transferred to Gene Screen Plus hybridized membranes (Du Pont, Stevenage, Hertfordshire). The cosmids were then screened for the presence of the prn gene encoding P. 70 by DNA : DNA hybridization using a radioactively labelled 1.8kb Cla1 restriction fragment isolated from the related prn gene from B.pertussis. The Cla1 fragment was gel purified (Tautz & Renz, Anal. Biochem. 132: 14-19, 1983) following digestion of cosmid pl69 (Charles et al Proc. Natl. Acad-Sci. USA 86:3554-3558, 1989). The fragment was nick translated with a kit supplied by BCL, Mannheim, and $[^{32}_{\alpha}P]$-ATP (Amersham), and hybridized with the B.parapertussis gene bank filters as previously described (Charles et al, J.Gen.Microbiol. 136: 353-358 (1990)). Three positive colonies were identified and one, harbouring cosmid pBD811 was selected for further analysis.

Subcloning and DNA sequencing

Restriction fragments of cosmid pBD811 were cloned in M13mp18 and M13mp19 (Yanisch-Perron et al, Gene 33:103-119, 1985) and sequenced using universal primer, $[^{35}_{\alpha}S]$ dATP (deoxyadenosine 5'- $[^{35}_{\alpha}S]$ thiotriphosphate) dideoxynucleotide triphosphates, and both gradient and wedge gels (Biggin et al, Proc.Natl. Acad.Sci USA 80:3963-3965, 1983; Sanger et al, Proc. Natl. Acad.Sci. USA 71:5463-5467, 1977). To resolve compression artifacts some clones were sequenced with modified T7 DNA polymerase (Tabor & Richardson, Proc.Natl. Acad.Sci USA 84:4767-4771, 1987) and 7-deaza-2'-dGTP (Mizusawa et al, Nucl.Acids.Res. 14: 1319-1324, 1986) using a kit supplied by Pharmacia. Gaps in the sequence were filled in using synthetic oligonucleotides as specific primers (Charles et al, Nucl.

Acids.Res. 14:2201-2213, 1986).

Oligonucleotides for use as specific sequencing primers were made on a SAM1 oligonucleotide synthesizer (Biolabs).

Computer analysis of the DNA sequence revealed an open reading frame capable of encoding a protein of 922 amino acids with a calculated molecular weight of 95,177.

Expression of P.70 antigen from B.parapertussis on the Surface of E.coli

Using Western blotting, it was not possible to detect the expression of P.70 in E.coli HB101 harbouring cosmid pBD811, although from DNA sequence analysis the cosmid was known to contain the entire structural gene. In order to express the B.parapertussis prn gene in E. coli, the expression vector pKK233-2 (Amann & Brosius, Gene 40:183-90, 1985) was used, which directs high level transcription from a trc promoter.

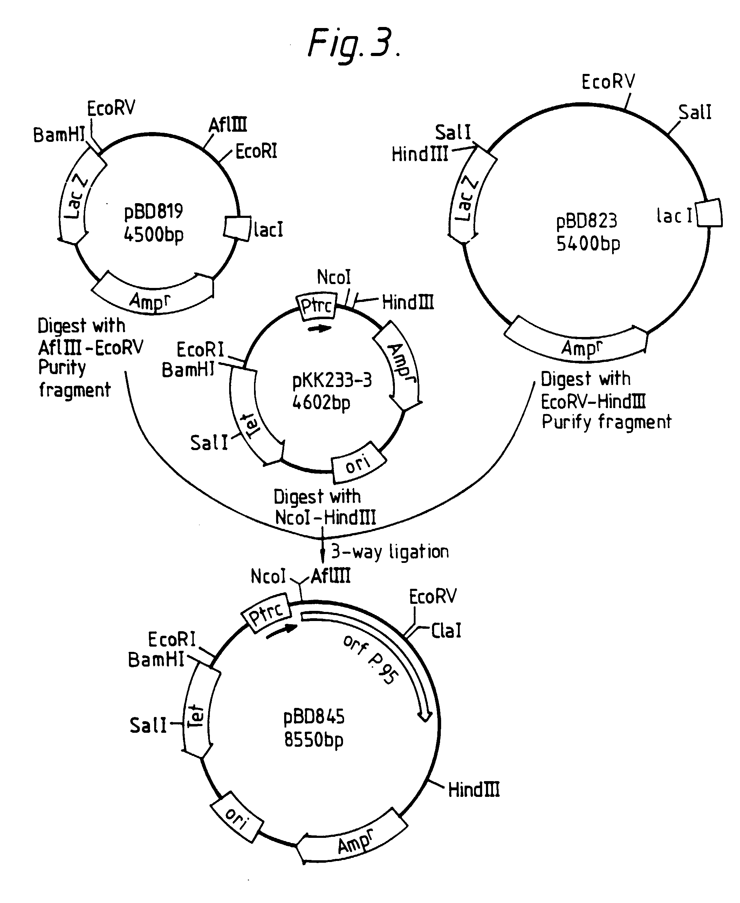

A three-way ligation using Ncol-HindIII digested pKK233-2; a 1.6kb AflIII-EcoRV fragment from the 5' end of P.70 prn and a 2.2 kb EcoRV-HindIII fragment containing the 3' end of P.70 prn was carried out (see Fig. 3). The ligation mix was used to transform E.coli TG1 and transformants expressing P.70 antigen identified by their ability to cross-react with the mAb BB05 (Novotny et al, Develop.Biol.Stand. 61:27-41, 1985) in protein dot blots. Colonies returning a positive signal were then isolated, and miniplasmid preps (Maniatis et al, Molecular Cloning: A labaratory Manual, Cold Spring Harbor, N.Y., 1982) carried out to verify that a full-length insert had been cloned. One of the colonies, harbouring plasmid pBD845, was selected for further study. SDS-PAGE electrophoresis was followed by Western blotting. Samples were transferred to nitrocellulose by the method of Towbin (Proc.Natl.Acad-Sci USA 76: 4350-4354, 1984). The detection system used was horse radish peroxidase conjugated goat anti-mouse IgG using 4-chloro-1-napthol as substrate (Fairweather et al, J.Bacteriol. 165:21-27, 1986). Western blotting of E.coli TG1 harbouring pBD845, (containing the entire structural gene for P.95), shows that a protein with a molecular weight of 95kDa (P.95) is weakly expressed in E. coli, along with a more significant band of 70kDa (P. 70). A large number of lower molecular weight species that cross-react with BB05 are also seen suggesting that P.70/P.95 is unstable in E.coli. These observations suggest that the P.95 form of the protein is initially expressed and is subsequently processed to the P.70 form.

Slide agglutination assays. Samples of E.coli strains harbouring pBD845 to be tested ($10^6$-$10^7$) were mixed with 30μl of IgG purified polyclonal rabbit anti-P. 69 antibody on a glass microscope slide and visually scored for clumping after 2 mins as compared with a negative control of either vir⁻ B.parapertussis or E.coli TG1. E.coli strains harbouring pBD845 can be agglutined, and this agglutination occurs just as rapidly as with vir⁺ B.parapertussis suggesting that recombinant P.70 protein is correctly expressed on the surface of E. coli.

**Claims**

1.  A protein which is uncontaminated by components from B.parapertussis, which is capable of binding to antibody which also binds the native P.70 antigen of B.parapertussis and which has (a) the amino acid sequence shown in Figure 1 from amino acid residue Asp 35 to Asn 643, or (b) an amino acid sequence which has a homology of more than 98% with the said amino acid sequence (a).

2.  A DNA sequence which encodes a protein as defined in claim 1.

3.  A DNA sequence according to claim 2, which consists essentially of the nucleotide sequence shown in Figure 1 from nucleotide 247 to nucleotide 2073.

4.  A DNA sequence according to claim 2, which differs from the nucleotide sequence shown in Figure 1 from nucleotide 247 to nucleotide 2073 at no more than twelve positions.

5.  A protein which has the amino acid sequence shown in Figure 1 or an amino acid sequence which has a homology of more than 98% with the amino acid sequence shown in Figure 1.

6.  A DNA sequence which encodes a protein as defined in claim 5.

7.  A DNA sequence according to claim 6, which consists essentially of the nucleotide sequence shown in Figure 1 from nucleotide 145 to nucleotide 2910.

8.  A DNA sequence according to claim 6, which differs from the nucleotide sequence shown in Figure 1 from nucleotide 145 to nucleotide 2910 at no more than twelve positions.

9.  A DNA sequence which consists essentially of the sequence shown in Figure 1 or a sequence which has a homology of more than 98% with the sequence shown in Figure 1.

10. An expression vector which contains a DNA sequence as defined in any one of claims 2 to 4 and 6 to 9, and which, when provided in a suitable host, is capable of expressing a protein as defined in claim 1 or 5.

11. A vector according to claim 10, which is a plasmid.

12. A host transformed with an expression vector as defined in claim 10 or 11.

13. A host according to claim 12, which is a strain of E. coli.

14. A process for the preparation of a protein as defined in claim 1, which process comprises maintaining a host transformed with an expression vector which contains a DNA sequence as defined in any one of claims 2 to 4 and which is capable of expressing the said protein in the said host, under such conditions that the said protein is expressed.

15. A process for the preparation of a protein as defined in claim 5, which process comprises maintaining a host transformed with an expression vector which contains a DNA sequence as defined in any one of claims 6 to 9 and which is capable of expressing the said protein in the said host, under such conditions that the said protein is expressed.

16. A pharmaceutical composition comprising a pharmaceutically acceptable carrier or diluent and, as active ingredient, a protein as defined in claim 1 or 5.

**Patentansprüche**

1.  Protein, welches nicht durch Komponenten von B. parapertussis verunreinigt ist, welches in der Lage ist, an einen Antikörper zu binden, der ebenfalls das native P.70-Antigen von B. parapertussis bindet, und welches (a) die in Figur 1 gezeigte Aminosäuresequenz vom Aminosäurerest Asp 35 bis Asn 643 oder (b) eine Aminosäuresequenz, welche eine Homologie von mehr als 98 % mit der Aminosäuresequenz (a) aufweist, besitzt.

2.  DNA-Sequenz, welche ein gemäß Anspruch 1 definiertes Protein codiert.

3.  DNA-Sequenz gemäß Anspruch 2, welche im wesentlichen aus der in Figur 1 gezeigten Nucleotidsequenz von Nucleotid 247 bis Nucleotid 2073 besteht.

4.  DNA-Sequenz gemäß Anspruch 2, welche sich von der in Figur 1 gezeigten Nucleotidsequenz von Nucleotid 247 bis Nucleotid 2073 an nicht mehr als zwölf Positionen unterscheidet.

5.  Protein, welches die in Figur 1 gezeigte Aminosäuresequenz oder eine Aminosäuresequenz besitzt, welche eine Homologie von mehr als 98 % mit der in Figur 1 gezeigten Aminosäuresequenz aufweist.

**6.** DNA-Sequenz, welche ein gemäß Anspruch 5 definiertes Protein codiert.

**7.** DNA-Sequenz gemäß Anspruch 6, welche im wesentlichen aus der in Figur 1 gezeigten Nucleotidsequenz von Nucleotid 145 bis Nucleotid 2910 besteht.

**8.** DNA-Sequenz gemäß Anspruch 6, welche sich von der in Figur 1 gezeigten Nucleotidsequenz von Nucleotid 145 bis Nucleotid 2910 an nicht mehr als zwölf Positionen unterscheidet.

**9.** DNA-Sequenz, welche im wesentlichen aus der in Fig. 1 gezeigten Sequenz oder aus einer Sequenz besteht, welche eine Homologie von mehr als 98 % mit der in Figur 1 gezeigten Sequenz aufweist.

**10.** Expressionsvektor, welcher eine DNA-Sequenz, wie sie nach einem der Ansprüche 2 bis 4 und 6 bis 9 definiert ist, enthält und welcher, wenn er in einem geeigneten Wirt vorliegt, in der Lage ist, ein gemäß Anspruch 1 oder 5 definiertes Protein zu exprimieren.

**11.** Vektor gemäß Anspruch 10, welcher ein Plasmid ist.

**12.** Wirt, welcher mit einem gemäß Anspruch 10 oder 11 definierten Expressionsvektor transformiert ist.

**13.** Wirt gemäß Anspruch 12, welcher ein *E.coli*-Stamm ist.

**14.** Verfahren zur Herstellung eines gemäß Anspruch 1 definierten Proteins, wobei das Verfahren das Halten eines Wirtes, der mit einem Expressionsvektor, welcher eine gemäß einem der Ansprüche 2 bis 4 definierte DNA-Sequenz enthält und in der Lage ist, das Protein in dem Wirt zu exprimieren, transformiert ist, unter solchen Bedingungen, daß das Protein exprimiert wird, umfaßt.

**15.** Verfahren zur Herstellung eines gemäß Anspruch 5 definierten Proteins, wobei das Verfahren das Halten eines Wirtes, der mit einem Expressionsvektor, welcher eine gemäß einem der Ansprüche 6 bis 9 definierte DNA-Sequenz enthält und in der Lage ist, das Protein in dem Wirt zu exprimieren, transformiert ist, unter solchen Bedingungen, daß das Protein exprimiert wird, umfaßt.

**16.** Pharmazeutische Zusammensetzung, umfassend einen/ein pharmazeutisch annehmbaren(s) Träger oder Verdünnungsmittel und, als aktiven Bestandteil, ein gemäß Anspruch 1 oder 5 definiertes Protein.

**Revendications**

**1.** Protéine qui n'est pas contaminée par des composants provenant de B.parapertussis, qui est capable de se lier à un anticorps qui se lie aussi à l'antigène P.70 natif de B.parapertussis et qui a (a) la séquence d'acides aminés représentée à la figure 1 depuis le résidu d'acide aminé Asp 35 jusqu'à Asn 643, ou (b) une séquence d'acides aminés qui a une homologie de plus de 98% avec ladite séquence d'acides aminés (a).

**2.** Séquence d'ADN qui code pour une protéine telle que définie dans la revendication 1.

**3.** Séquence d'ADN selon la revendication 2, qui consiste essentiellement en la séquence nucléotidique représentée à la figure 1 depuis le nucléotide 247 jusqu'au nucléotide 2 073.

**4.** Séquence d'ADN selon la revendication 2, qui diffère de la séquence nucléoditique représentée à la figure 1 depuis le nucléotide 247 jusqu'au nucléotide 2 073 en pas plus de douze positions.

**5.** Protéine qui a la séquence d'acides aminés représentée à la figure 1 ou une séquence d'acides aminés qui a une homologie de plus de 98% avec la séquence d'acides aminés représentée à la figure 1.

**6.** Séquence d'ADN qui code pour une protéine telle que définie dans la revendication 5.

**7.** Séquence d'ADN selon la revendication 6, qui consiste essentiellement en la séquence nucléotidique représentée à la figure 1 depuis le nucléotide 145 jusqu'au nucléotide 2 910.

**8.** Séquence d'ADN selon la revendication 6, qui diffère de la séquence nucléotidique représentée à la figure 1 depuis le nucléotide 145 jusqu'au nucléotide 2 910 en pas plus de douze positions.

**9.** Séquence d'ADN qui consiste essentiellement en la séquence représentée à la figure 1 ou une séquence qui a une homologie de plus de 98% avec la séquence représentée à la figure 1.

**10.** Vecteur d'expression qui contient une séquence d'ADN telle que définie dans l'une quelconque des revendications 2 à 4 et 6 à 9, et qui, lorsque il est introduit dans un hôte approprié, est capable d'exprimer une protéine telle que définie dans la revendication 1 ou 5.

**11.** Vecteur selon la revendication 10, qui est un plasmide.

**12.** Hôte transformé avec un vecteur d'expression tel que défini dans la revendication 10 ou 11.

**13.** Hôte selon la revendication 12, qui est une souche d'*E.coli.*

**14.** Procédé pour la préparation d'une protéine telle que définie dans la revendication 1, procédé qui comprend l'étape consistant à maintenir un hôte transformé avec un vecteur d'expression qui contient une séquence d'ADN telle définie dans l'une quelconque des revendications 2 à 4 et qui est capable d'exprimer ladite protéine dans ledit hôte, dans des conditions telles que ladite protéine soit exprimée.

**15.** Procédé pour la préparation d'une protéine telle que définie dans la revendication 5, procédé qui comprend l'étape consistant à maintenir un hôte transformé avec un vecteur d'expression qui contient une séquence d'ADN telle que définie dans l'une quelconque des revendications 6 à 9 et qui est capable d'exprimer ladite protéine dans ledit hôte, dans des conditions telles que ladite protéine soit exprimée.

**16.** Composition pharmaceutique comprenant un support ou un diluant pharmaceutiquement acceptable et, en tant qu'ingrédient actif, une protéine telle que définie dans la revendication 1 ou 5.

# Fig.1.

```
ATCGAATGATGCGTCGCTGTAACACGACGAAATACCGTGCAGCGGTTCGTAGGTTCGTCGCCGCCATTCTTCCCTGTTCCATCCCGGTGCCGGGCCATGGCGGG
        10        20        30        40        50        60        70        80        90       100       110       120

            M  M  S  L  B  R  I  V  K  A  A  P  L  R  R  T  I  L  A  M  A  L  G  A  L  Q  A  A  P  A  R  A
CGTCTGCTTCACCCGGCCATCCAATGAACCATGTCTGTCTGTCACGGCATTGTCAAGGCGGCCCCGCTGGCCCGCCGCATGGCCGCCTGGGCCGCCCGCCCCGGCCGCG
       130       140       150       160       170       180       190       200       210       220       230       240

  Y  A  D  W  M  M  Q  B  I  I  K  A  G  E  R  Q  M  G  I  M  I  K  Q  S  Q  G  A  G  V  R  T  A  T  G  T  T  I  K  V  S
TACGGCGACTGGAACAACCAGTCCATCATCAAGGCCGGAGCGGCCGAGCGACGCATCCACATCAAGCAAGGCGATGGCGTACGGACCGCCACCGGAACGGACCATCAAGGTAAGC
       250       260       270       280       290       300       310       320       330       340       350       360

  Q  R  Q  A  Q  D  V  L  L  E  N  P  A  A  E  L  R  F  Q  N  G  S  V  T  S  S  G  Q  L  F  D  E  G  V  R  R  F  L  G  T
GGTCGGTCAGGCCCCCAGGGCGTCCTGCTGGAAAATCCCGGCGCCGAGCTGCGGTTCCAGAACGGCAGCGTCACGTCTTCGGGACAGCTGTTCGACGAAGGGCGTCCGGCGCTTTCTGGGCACC
       370       380       390       400       410       420       430       440       450       460       470       480

  V  T  V  K  A  G  K  L  V  A  D  M  A  T  L  A  N  V  S  D  T  R  D  D  D  G  I  A  L  V  V  A  G  E  Q  A  D  A  S  I
GTCACCGTCAAGGCCGGCAAGCTGGTCGCCGATATGGCCACGCTCGCCAACGTCAGCGGCGACACCCGGGACGACGAGGGCATCGCGCTCTATGTGGCCGGCGAGCAGGCCGAGCCAGCCATC
       490       500       510       520       530       540       550       560       570       580       590       600

  A  D  S  T  L  Q  G  A  G  Q  V  R  V  E  R  G  A  N  V  T  V  Q  R  S  T  I  V  D  G  G  L  M  I  G  V  L  D  P  L  Q
GCCGACAGCACCCTTGCAGGGGCCGGGCAGGTGCGGGTCGGGGCCAATGTCACGGTTGCACGTCGTTGACGCACCATCGTTGACGGGGGCCTGATGATCGGCATATCGCAGCCCTGCAGCCGGTGCAG
       610       620       630       640       650       660       670       680       690       700       710       720

  P  E  D  L  P  P  S  R  V  V  L  G  D  T  S  V  I  A  V  P  A  S  G  A  P  N  A  V  F  V  F  G  A  N  E  L  T  V  D  G
CCGGAAGACCTTCCCGCCCAGTCGTGGTGCTGGGCGACACCAGCGTGACCGCCGTTCCCGCCGGCGGCGGTGTTGTTTGTATTCGGGGCCAATGAGCTTACGGTTGATGGC
       730       740       750       760       770       780       790       800       810       820       830       840

  Q  M  I  T  D  G  R  A  A  G  V  A  A  M  D  G  A  I  V  M  L  Q  R  A  T  I  R  G  D  A  P  A  G  G  A  V  P  G  G
GGGCACATCACCGGGGGCGGGCGGGCAGCGGGGCCATGGACGGGGCGATCGTGCATCTGCAGCGGGCAGCGGGGACGGGGCGGCCGCGGGGGGTGCCGGGTTCCAGGCGGGT
       850       860       870       880       890       900       910       920       930       940       950       960

  A  V  P  G  G  A  V  P  D  D  F  G  F  L  L  D  G  W  V  G  V  D  V  S  D  S  T  V  D  L  A  Q  S  I  V  E  A  P  D  L
GCCGGTTCCCGGCGGCGGTGCCGGTTCCGGGCGGCGCCCCTCCTTGACGGCTGGGTAGGCGTGGATGTATCGGACTCCAGTCGATCGCGCTCAGTGTCGATCTGGCCCAGTCGATCGTCGAGGCGCCCAAGCTG
       970       980       990      1000      1010      1020      1030      1040      1050      1060      1070      1080
```

9

# Fig.1 Cont (1).

```
         A  A  I  R  A  G  R  G  A  R  V  T  V  S  G  G  S  L  S  A  P  H  G  N  V  I  E  T  G  G  G  A  R  R  R  F  P  P  P  A
GGCGCCGCGATCCGGGCGGGCCGCGGCGCCAGGGTGACGGTGTCGGGCGGCAGCTTGTCCGCACCGCACGGCAATGTCATCGAGACCGGCGGCGGTGCGCGTCGCTTCCCGCCTCCGGCC
         1090       1100       1110       1120       1130       1140       1150       1160       1170       1180       1190       1200


   S  P  L  S  I  T  L  G  A  G  A  R  A  G  G  R  A  L  L  Y  R  V  L  P  E  P  V  K  L  T  L  A  G  G  A  G  G  G  G  G
TCGCCCCTGTCGATCACCTTGCAGGCGGGCGCACGGGCGCAGGGGAGGGCGCTGCTGTACCGGGTCCTGCCGGAGCCCGTGAAGCTGACGCTGGCGGGCGGCGCCCAGGGGCAGGGCGAC
         1210       1220       1230       1240       1250       1260       1270       1280       1290       1300       1310       1320


   I  V  A  T  E  L  P  P  I  P  G  A  S  S  G  P  L  D  V  A  L  A  S  G  A  R  M  T  G  A  T  R  A  V  D  S  L  S  I  D
ATCGTCGCGACGGAGCTGCCTCCCATTCCAGGCGCGTCGAGCGGGCCGCTCGACGTGGCGCTGGCCAGCCAGGCCCGATGGACGGGCGCTACCCGCGCGGTCGACTCGCTGTCCATCGAC
         1330       1340       1350        .1360      1370       1380       1390       1400       1410       1420       1430       1440


   N  A  T  W  V  M  T  D  N  S  N  V  G  A  L  R  L  A  S  D  G  S  V  D  F  G  G  P  A  E  A  G  R  F  K  V  L  M  V  D
AACGCCACCTGGGTCATGACGGACAACTCGAACGTCGGCGCGCTGCGGCTGGCCAGCGACGGCAGCGTCGATTTCCAGCAGCCGGCCGAAGCTGGGCGGTTCAAGGTCCTGATGGTCGAT
         1450       1460       1470       1480       1490       1500       1510       1520       1530       1540       1550       1560


   T  L  A  G  S  G  L  F  R  M  N  V  F  A  D  L  G  L  S  D  K  L  V  V  M  R  D  A  S  G  D  H  R  L  M  V  R  N  S  G
ACGCTGGCGGGTTCGGGGCTGTTCCGCATGAATGTCTTCGCGGACCTGGGGCTGAGCGACAAGCTGGTCGTCATGCGGGACGCCAGCGGCCAGCACAGGCTGTGGGTCCGCAACAGCGGC
         1570       1580       1590       1600       1610       1620       1630       1640       1650       1660       1670       1680


   S  E  P  A  S  G  N  T  M  L  L  V  D  T  P  R  G  S  A  A  T  F  T  L  A  N  K  D  G  K  V  D  I  G  T  Y  R  Y  R  L
AGCGAGCCGGCCAGCGGCAACACCATGCTGCTGGTGCAGACGCCACGAGGCAGCGCGGCGACCTTTACCCTTGCCAACAAGGACGGCAAGGTCGATATCGGTACCTACCGCTATCGATTG
         1690       1700       1710       1720       1730       1740       1750       1760       1770       1780       1790       1800


   A  A  N  G  N  G  D  W  S  L  V  G  A  K  A  P  P  A  P  K  P  A  P  G  P  G  P  G  P  G  P  G  P  P  G  P  P  G  P  P
GCCGCCAACGGCAATGGGCAGTGGAGCCTGGTGGGCGCGAAGGCGCCGCCGGCGCCCAAGCCCGCGCCGCAGCCCGGTCCCCAGCCCGGTCCCCAGCCGCCGCAGCCGCCGCAGCCGCCG
         1810       1820       1830       1840       1850       1860       1870       1880       1890       1900       1910       1920


   G  P  P  G  P  P  G  P  P  G  R  G  P  E  A  P  A  P  G  P  G  P  P  A  G  R  E  L  S  A  A  A  N  A  A  V  N  T  G  G  V  G
CAGCCGCCGCAGCCGCCGCAGCCGCCCACAGAGGCAGCCGGAAGCGCCGGCGCCGCAACCGCCGGCGGGCAGGGAGTTGTCCGCCGCCGCCAACGCGGCGGTCAACACGGGTGGGGTGGGC
         1930       1940       1950       1960       1970       1980       1990       2000       2010       2020       2030       2040


   L  A  S  T  L  W  V  A  E  S  N  A  L  S  K  R  L  G  E  L  R  L  N  P  D  A  G  G  A  W  G  R  G  F  A  G  R  R  G  L
CTGGCCAGCACGCTCTGGTACGCCGAAAGCAATGCGTTGTCCAAGCGCCTGGGCGAGTTGCGCCTGAATCCGGACGCCGGCGGCGCTTGGGGCCGCGGCTTCGCGCAACGCCAGCAACTG
         2050       2060       2070       2080       2090        2100       2110       2120       2130       2140       2150       2160
```

EP 0 573 435 B1

# Fig.1 Cont (2).

EP 0 573 435 B1

```
D  N  R  A  G  R  R  R  F  D  Q  K  V  A  G  F  E  L  G  A  D  H  A  V  A  V  A  G  G  R  M  H  L  G  G  L  A  G  Y  T  R
GACAACCGCGCCGGGCGGCGCTTCGACCAGAAGGTGGCCGGCTTCGAGCTGGGCGCCGACCACGCGGTGGCGGTGGCCGGCGGGCGCTGGCACCTGGGCGGGCTGGCCGGCTATACGCGC
    2170      2180      2190      2200      2210      2220      2230      2240      2250      2260      2270      2280

G  D  R  G  F  T  D  D  G  G  G  H  T  D  S  V  H  V  G  G  Y  A  T  Y  I  A  N  S  G  F  V  L  D  A  T  L  R  A  S  R
GGCGACCGCGGCTTTACCGGCGACGGCGGCGGCCACACCGACAGCGTGCATGTCGGGGGCTATGCCACCTATATCGCCAACAGCGGTTTCTACCTGGACGCGACGCTGCGCGCCAGCCGC
    2290      2300      2310      2320      2330      2340      2350      2360      2370      2380      2390      2400

L  E  N  D  F  K  V  A  G  S  D  G  Y  A  V  K  G  K  Y  R  T  H  G  V  G  V  S  L  E  A  G  R  R  R  F  A  H  A  D  G  W
CTCGAAAATGACTTCAAGGTGGCGGGCAGCGATGGGTACGCGGTCAAGGGCAAGTACCGCACCCATGGGGTAGGCGTCTCGCTCGAGGCGGGCCGGCGGCTTCGCCCATGCCGACGGCTGG
    2410      2420      2430      2440      2450      2460      2470      2480      2490      2500      2510      2520

F  L  E  P  D  A  E  L  A  V  F  R  ,V  G  D  G  A  V  R  A  A  N  G  L  A  V  R  D  E  G  G  S  S  V  L  G  R  L  G  L
TTCCTCGAGCCCGCAGGCCGAGCTGGCGGTGTTCCGGGTCGGCGGCGGTGCGTACCGCGCGGCCAATGGCCTGCGGGTGCGCGACGAAGGCGGCAGCTCGGTGCTGGGTCGCCTGGGCCTG
    2530      2540      2550      2560      2570      2580      2590      2600      2610      2620      2630      2640

E  V  G  K  R  I  E  L  A  G  D  R  D  V  Q  P  V  I  K  A  S  V  L  D  E  F  D  G  A  G  T  V  R  T  N  G  I  A  H  R
GAGGTCGGCAAGCGCATCGAACTGGCAGGCGGCAGGCAGGTGCAGCCATACATCAAGGCCAGCGTGTTGCAGGAGTTCGACGGCGCGGGGTACGGTACGCACCAACGGCATCGCGCATCGC
    2650      2660      2670      2680      2690      2700      2710      2720      2730      2740      2750      2760

T  E  L  R  G  T  R  A  E  L  G  L  G  H  A  A  A  L  G  R  G  H  S  L  Y  A  S  V  E  Y  S  K  G  P  K  L  A  H  P  W
ACCGAACTGCGCGGCACGCGCGCCGAACTGGGCCTGGGCATGGCCGCCGCGCTGGGCCGCGGCCACAGCCTGTATGCCTCGTACGAGTACTCCAAGGGCCCCGAAGCTGGCCATGCCGTGG
    2770      2780      2790      2800      2810      2820      2830      2840      2850      2860      2870      2880

T  F  H  A  G  Y  R  Y  S  W  *
ACCTTCCACGCGGGCTACCGGTACAGCTGGTAAAGCGAGAAGGGTCCATCCCCCGCGGAGGAGTTTTTCCTGGAGGTTGGCCGGTGCCAGTCTCCAGGCTCAGGCGGCCAGGGCCTGCGG
    2890      2900      2910      2920      2930      2940      2950      2960      2970      2980      2990      3000
```

EP 0 573 435 B1

Fig.2.

# Fig.3.